# EUROPEAN PATENT APPLICATION

(11) **EP 4 725 600 A1**
(43) Date of publication of application: **15.04.2026**
(21) Application number: 24306679.2
(22) Date of filing: 11.10.2024
(51) Int. Cl.: B01L 3/00, B01L 7/00, C12Q 1/68

(54) **NUCLEIC ACID AMPLIFICATION IN DROPLETS**

(71) Applicant: Hajji, Ismail, 75006 Paris (FR)
(72) Inventor: Hajji, Ismail, 75006 Paris (FR)
(74) Representative: Bandpay & Greuter

(57) **Abstract**

The invention relates to a device for nucleic acid amplification, comprising: a channel; a temperature regulation system; a droplet manipulation mechanism configured to flow a droplet along the channel; wherein: the channel comprises portions in contact with heating zones of the temperature regulation system alternating with portions in contact with cooling zones of the temperature regulation system, the heating zones being configured to be at a higher temperature than the cooling zones, and wherein: each heating zone comprises an upstream section and a downstream section relative to the direction of flow in the channel, the downstream section is configured to be at a uniform temperature T_{H}, the upstream section is configured to be at a temperature which is higher than T_{H}; and/or: each cooling zone comprises an upstream section and a downstream section relative to the direction of flow in the channel, the downstream section is configured to be at a uniform temperature Tc, the upstream section is configured to be at a temperature which is lower than Tc.

## Description

### Technical field

The present invention relates to a device and method for nucleic acid amplification.

### Technical background

Polymerase chain reaction, or PCR, is a major technique in molecular biology, used for nucleic acid amplification. Generally, PCR comprises a series of thermal cycles, each cycle composed of 3 steps: denaturation of double-stranded nucleic acids into single-stranded nucleic acids; annealing of oligonucleotide primers to the single-stranded nucleic acids; and elongation of the primers by a DNA polymerase. Each step is carried out within a specific range of temperatures. Generally, denaturation can occur at a temperature of from 90°C to 95°C; annealing can occur at a temperature of from 50°C to 65°C; and elongation by the polymerase can occur at a temperature of from 50°C to 72°C. Since many polymerases are highly active in the temperature range for primer annealing, the annealing and elongation steps are often combined into a single step (annealing-elongation step). This two-step PCR (denaturation step and annealing-elongation step) can significantly reduce the run time, compared to the conventional three-step PCR (denaturation, annealing, and elongation steps).

Several types of PCR protocols are widely used in diagnostics and clinical settings, such as quantitative PCR (qPCR, also known as real-time PCR) in which the amount of amplified product is quantified; reverse transcription PCR (RT-PCR) for RNA analysis; and digital PCR (dPCR) in which a sample including a target nucleic acid is segmented in a small compartment (usually a few nanoliters).

Nowadays, droplet microfluidics is well integrated with PCR protocols, such as droplet-digital PCR (ddPCR), in which a single molecule of the target nucleic acid is encapsulated in a droplet and amplified through thermal cycles.

The integration of PCR protocols with droplet microfluidics enhances the accuracy, sensitivity, and reliability of the results, making it a powerful tool for both research and clinical applications, especially in cancer diagnostics.

US patent application US2013065241A1 discloses a composition and a method for amplification and detection of nucleic acid sequences based on continuous flow thermal gradient PCR.

However, the above method is insufficient for optimizing PCR efficiency because the thermal gradient oscillates and thus a steady state necessary for efficient PCR reactions is not reached. Additionally, the volume capacity is quite limited.

The article I. Hajji, et al., Sens. Actuators B Chem. 303, 127171 (2020) discloses a fully automated droplet microfluidics platform for RT-qPCR that couples a specifically designed thermal system with a fluorescence excitation/detection module.

It has been found by the present inventors that the above platform may still exhibit insufficient amplification efficiency because the reaction rates of the PCR steps (denaturation, annealing and elongation or annealing-elongation) are constrained by the time required for the temperature transition from one PCR step to the subsequent PCR step.

Thus, there is still a need for an improved device and method for nucleic acid amplification.

### Summary of the invention

It is a first object of the invention to provide a device for nucleic acid amplification, comprising: a channel; a temperature regulation system; a droplet manipulation mechanism configured to flow a droplet along the channel; wherein: the channel comprises portions in contact with heating zones of the temperature regulation system alternating with portions in contact with cooling zones of the temperature regulation system, the heating zones being configured to be at a higher temperature than the cooling zones, and wherein: each heating zone comprises an upstream section and a downstream section relative to the direction of flow in the channel, the downstream section is configured to be at a uniform temperature T_{H}, the upstream section is configured to be at a temperature which is higher than T_{H}; and/or: each cooling zone comprises an upstream section and a downstream section relative to the direction of flow in the channel, the downstream section is configured to be at a uniform temperature Tc, the upstream section is configured to be at a temperature which is lower than Tc.

In some embodiments, the upstream section of each heating zone is configured to be at a uniform temperature higher than T_{H}; and/or the downstream section of each cooling zone is configured to be at a uniform temperature lower than Tc.

In some embodiments, the upstream section of each heating zone is configured so that the temperature in this upstream section gradually or incrementally decreases in the upstream to downstream direction; and/or the upstream section of each cooling zone is configured so that the temperature in this upstream section gradually or incrementally increases in the upstream to downstream direction.

In some embodiments, the channel is coiled.

In some embodiments, the temperature regulation system comprises a plurality of thermoelectric modules and one or more heat spreading elements on one side of the thermoelectric modules.

In some embodiments, the temperature regulation system comprises a heating module comprising all heating zones and a cooling module comprising all cooling zones, wherein the heating zones are preferably arranged along a same planar area of the heating module and the cooling zones are preferably arranged along a same planar area of the cooling module.

In some embodiments, the temperature regulation system comprises: different thermoelectric modules for controlling the temperature of the upstream sections and downstream sections in the heating zones, wherein, preferably, these different thermoelectric modules are arranged in parallel, and extend in a direction perpendicular to the direction of flow in the channel; and/or different thermoelectric modules for controlling the temperature of the upstream sections and downstream sections in the cooling zones, wherein, preferably, these different thermoelectric modules are arranged in parallel, and extend in a direction perpendicular to the direction of flow in the channel.

In some embodiments, the temperature regulation system comprises a single downstream heating thermoelectric module for controlling the temperature of the downstream sections of all heating zones; and/or a single downstream cooling thermoelectric module for controlling the temperature of the downstream sections of all cooling zones.

In some embodiments, the temperature regulation system comprises one or more upstream heating thermoelectric modules for controlling the temperature of the upstream sections of all heating zones; and/or one or more upstream cooling thermoelectric modules for controlling the temperature of the upstream sections of all cooling zones.

In some embodiments, in each heating zone, the ratio of the length of the upstream section to the length of the downstream section, along the direction of flow in the channel, is from 1:2 to 1:50, preferably from 1:3 to 1:20, more preferably from 1:4 to 1:10; and/or in each cooling zone, the ratio of the length of the upstream section to the length of the downstream section, along the direction of flow in the channel, is from 1:2 to 1:50, preferably from 1:3 to 1:20, more preferably from 1:4 to 1:10.

In some embodiments, the temperature regulation system is configured to adjust a temperature profile of the upstream sections of the heating zones and/or a temperature profile of the upstream sections of the cooling zones as a function of the flow rate in the channel.

It is a second object of the invention to provide a method of amplifying a nucleic acid using the above-described device for nucleic acid amplification, comprising: providing at least one droplet containing a nucleic acid and reagents for amplifying said nucleic acid, and flowing the droplet along the channel and alternately heating and cooling the droplet as the droplet flows through portions in contact with heating zones and portions in contact with cooling zones.

In some embodiments, as the droplet flows through a portion in contact with a heating zone, the temperature of the droplet varies and then remains constant; and/or as the droplet flows through a portion in contact with a cooling zone, the temperature of the droplet varies and then remains constant.

In some embodiments, the temperature of the droplet gradually or incrementally increases in the upstream to downstream direction and then remains constant as the droplet flows through a portion in contact with a heating zone; and/or the temperature of the droplet gradually or incrementally decreases in the upstream to downstream direction and then remains constant as the droplet flows through a portion in contact with a cooling zone.

In some embodiments, as the droplet flows through a portion in contact with a heating zone, the ratio of the duration for which the temperature of the droplet varies and is not constant to the total duration for which the droplet flows through the portion in contact with a heating zone is from 1 to 60%, preferably from 2 to 50%, more preferably from 5 to 40%, and most preferably from 10 to 30%; and/or as the droplet flows through a portion in contact with a cooling zone, the ratio of the duration for which the temperature of the droplet varies and is not constant to the total duration for which the droplet flows through the portion in contact with a cooling zone is from 1 to 60%, preferably from 2 to 50%, more preferably from 5 to 40%, and most preferably from 10 to 30%.

The present invention makes it possible to address the need expressed above. In particular, the invention provides a more efficient device and method for nucleic acid amplification.

This is achieved by a device for nucleic acid amplification, comprising a channel, a droplet manipulation mechanism, and a temperature regulation system, wherein heating zones of the temperature regulation system are at a higher temperature than cooling zones of the temperature regulation system, and wherein each heating zone comprises an upstream section and a downstream section relative to the direction of flow in the channel, the downstream section is at a uniform temperature T_{H}, and the upstream section is at a temperature which is higher than T_{H}; and/or wherein each cooling zone of the temperature regulation system comprises an upstream section and a downstream section relative to the direction of flow in the channel, the downstream section is at a uniform temperature Tc, and the upstream section is at a temperature which is lower than Tc.

In such a configuration, when a temperature transition occurs in a thermal cycle, it is possible to reduce the time required for a droplet to reach the uniform target temperature of each zone. More specifically, the uniform temperature T_{H} in the downstream section of each heating zone can be reached faster because the upstream section is at a temperature higher than T_{H}. Alternatively or additionally, the uniform temperature Tc in the downstream section of each cooling zone can be reached faster because the upstream section is at a temperature lower than Tc.

In the present invention, the uniform temperature of the downstream sections in heating and cooling zones can correspond to the temperature appropriate for a PCR step (for example, the uniform temperature in the downstream sections of the heating zones corresponds to the temperature at which denaturation occurs, and the uniform temperature in the downstream sections of the cooling zones corresponds to the temperature at which annealing-elongation occurs).

Thus, when nucleic acid amplification is performed using the device of the invention, the PCR reactions are minimally constrained by the thermalization time (time required for reaching the uniform temperature), if at all, resulting in an efficient nucleic acid amplification.

### Brief description of the drawings

**Figure 1** schematically illustrates an embodiment of the device of the invention. The dotted lines indicate an enlarged view of a cooling zone of a temperature regulation system of the device.
**Figure 2** schematically illustrates a heating zone of a temperature regulation system of another embodiment of the device of the invention.
**Figure 3** schematically illustrates a heat spreading element of the device of the invention as can be used for example in the device of **Figure 1****.** **Figure 3(a)** shows a top view of the heat spreading element, and **Figure 3(b)** shows a side cross-section of the heat spreading element, together with an enlarged view.
**Figure 4** schematically illustrates still another embodiment of the device of the invention.
**Figure 5** schematically illustrates the comb-like structure of the temperature regulation system of an embodiment of the device of **Figure 4****,** with an enlarged view of the structure in the solid-line rectangle.
**Figure 6** schematically illustrates an embodiment of a droplet manipulation mechanism which may be part of the device of the invention.
**Figure 7** schematically illustrates an embodiment of an RT module used in a device of the invention.

### Detailed description

The invention will now be described in more detail without limitation in the following description.

The term *"nucleic acid'* as used herein means a polymer composed of nucleotides, e.g. deoxyribonucleotides or ribonucleotides, more particularly deoxyribonucleotides.

The term *"channel"* as used herein means an elongated space such as a tube, duct, pipe, or conduit, along which fluids can flow. The channel is delimited by at least one inlet and at least one outlet. Each of the inlet and outlet may correspond to a connection port or may represent a mere junction with another channel for other upstream/downstream microfluidic operations.

The terms *"upstream"* and *"downstream"* as used herein refer to the location relative to the flow direction of a fluid. For example, an *"upstream section"* and a *"downstream section"* mean that the upstream section is located before the downstream section in the flow direction. Likewise, *"A is present upstream of B"* means that A is located before B in the flow direction, and *"A is present downstream of B"* means that A is located after B in the flow direction.

By *"uniform (temperature)"* is meant that the temperature remains stable throughout a specified area or zone, with minor allowable variations. For example, the uniform temperature can vary or fluctuate within a narrow range around an average value, such as ±1°C, preferably ±0.5°C, more preferably ±0.2°C.

By *"constant (temperature)"* is meant that the temperature remains stable over time, with minor allowable variations. For example, the constant temperature can vary or fluctuate within a narrow range around an average value, such as ±1°C, preferably ±0.5°C, more preferably ±0.2°C.

### Device for nucleic acid amplification

The device of the invention comprises a channel; a temperature regulation system; and a droplet manipulation mechanism configured to flow a droplet along the channel, preferably configured to simultaneously flow multiple droplets along the channel.

In some embodiments, the device may be a microfluidic device, a millifluidic device, or nanofluidic device.

The term *"microfluidic"* herein means a device or chip in which the minimal channel dimensions are of the order of 1 to less than 1000 µm. The term *"millifluidic"* herein means a device or chip in which the minimal channel dimensions are of the order of 1 to 10 mm. The term *"nanofluidic"* herein means a device or chip in which the minimal channel dimensions are of the order of less than 1 µm.

When the device is in use, a main fluid may flow along the channel, and droplets may be carried by the flow of main fluid. The main fluid may be a liquid, especially an oil, and more particularly a fluorinated oil, a mineral oil, a vegetal oil. The droplets may be made of a liquid which is immiscible with the main fluid, such as an aqueous solution. One or more surfactants may be present in the main fluid and/or in the droplets.

The droplet manipulation mechanism may comprise any conventional flow regulator, which may include one or more syringe pumps and valves, such as pinch valves, in order to adjust the flow rate of the fluid containing droplets in the channel.

The channel may be a capillary tube.

In some embodiments, the channel may have a substantially constant cross-section along its length.

In other embodiments, the channel may have a varying cross-section along its length.

The channel may have an internal diameter from 0.01 mm to 10 mm, preferably from 0.2 mm to 2 mm. If the cross-section of the channel is not circular, the diameter refers to the maximum dimension of the cross-section.

When the channel has a varying cross-section along its length, the diameters within the varying cross-section may fall within the above diameter range. For example, the channel may have a varying diameter along its length. Preferably, the channel has its smallest diameter at the inlet, with the diameter gradually increasing along its entire length. The channel having such a tapered diameter (gradually expanding diameter) may be advantageous for further accelerating the thermalization.

The channel may have a total length from 10 µm to 30 cm, preferably from 500 µm to 10 cm

The channel may be made of any suitable material. Especially for quantitative amplification (which will be explained later), it may be made of a material that allows for the visualization of the droplets which flow in the channel, such as a fluorinated polyolefin (e.g., polytetrafluoroethylene, fluorinated ethylene propylene), polydimethylsiloxane, cyclic olefin copolymer, silicone, or glass.

The internal cross-section of the channel may take any suitable shape, for example, circular, rectangular, square, triangular, oval, elliptical, or hexagonal shape. The internal cross-section of the channel is preferably circular.

In some embodiments, the channel may be transparent, semi-transparent, or partially transparent.

In some embodiments, the channel may be linear.

Alternatively or additionally, the channel may be flexible (can be curved, bended or deformed). For example, the channel may be curved in a form of serpentine or sinusoid, a flat spiral-shaped, or coiled.

In the device of the invention, the channel comprises portions in contact with heating zones of the temperature regulation system alternating with portions in contact with cooling zones of the temperature regulation system, the heating zones being at a higher temperature than the cooling zones. The channel may further comprise intermediate portions between the portions in contact with cooling zones and the portions in contact with heating zones.

The terms *"heating (zones)"* and *"cooling (zones)"* refer to the fact that, when the device is in use, the heating zones are hotter than the cooling zones. More specifically, in the alternation between heating zones and cooling zones along the channel, the temperature of each heating zone is greater than the temperature of the cooling zone that is present upstream of the heating zone, and/or the temperature of each cooling zone is lower than the temperature of the heating zone that is present upstream of the cooling zone. The above definition similarly applies when the cooling zones comprise first cooling zones and second cooling zones (which will be explained in more detail below).

In some embodiments, the channel comprising portions in contact with heating zones and cooling zones is substantially planar. For example, it can be segmented and/or curved. It may extend along a longitudinal axis and cross the longitudinal axis at multiple locations, so that there are portions on one side of the longitudinal axis and other portions on the opposite site of the longitudinal axis. The channel may have a substantially periodical shape, such as a crenelated or zigzag shape or a serpentine or sinusoid shape. The portions in contact with heating zones and the portions in contact with cooling zones may be arranged across opposite sides of the longitudinal axis.

Otherwise, the channel comprising portions in contact with heating zones and cooling zones may not lie in a single plane.

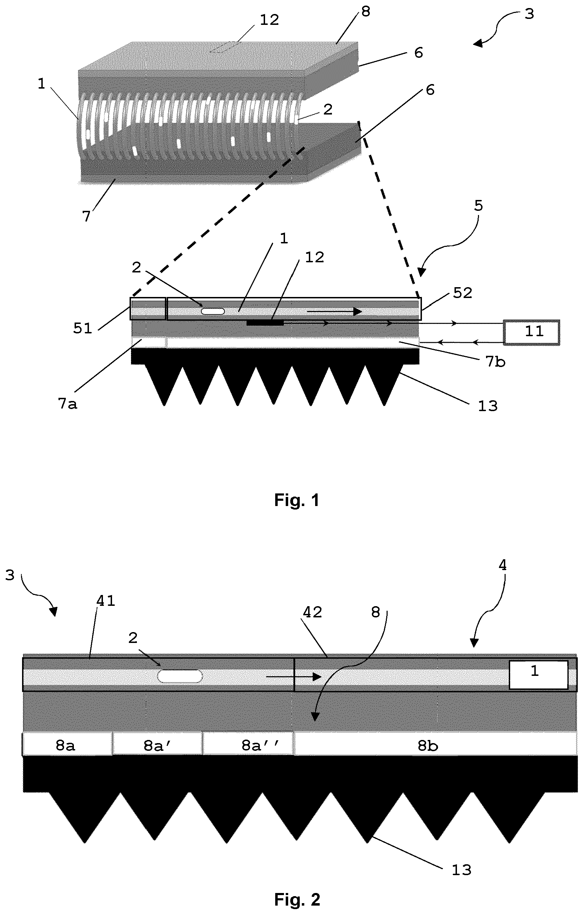

In preferred embodiments, the channel comprising portions in contact with heating zones and cooling zones is coiled, as shown in **Fig. 1****.**

The channel comprising portions in contact with heating zones and cooling zones may be coiled with 10 to 100 loops, preferably 15 to 60 loops (coils), more preferably 20 to 50 loops (coils). Such a coiled channel may extend three-dimensionally along a central axis. All loops may have substantially the same shape, *i*.*e.* each loop may be geometrically obtained from an adjacent loop by a translation along the central axis. The distance between two adjacent loops may be constant along the channel. The coiled channel may be a helical channel. Alternatively, each loop may be substantially contained within a plane, preferably perpendicular to the central axis, one minor part of the loop ensuring the connection with an adjacent loop. Alternatively, each loop may comprise a plurality of linear segments. Alternatively, each loop may comprise a plurality of curved segments, each curved segment contained within a plane.

The distance between adjacent loops may be from 0.01 mm to 5 mm, preferably from 0.1 mm to 1 mm.

In some embodiments, the portions of the channel in contact with the heating and/or cooling zones may be partially in contact with the heating and/or cooling zones.

In some embodiments, the portions of the channel in contact with the heating and/or cooling zones may be entirely in contact with the heating and/or cooling zones.

In some embodiments, the channel may comprise further portions, which are not in contact with heating zones and/or cooling zones, and which may be for example exposed to the exterior environment (and/or may be in contact with other elements than the heating zones and cooling zones). Such non-contact portions of the channel may be present between the portions in contact with the heating zones and portions in contact with the cooling zones.

In the device of the invention, each heating zone associated with a portion of the channel comprises an upstream section and a downstream section relative to the direction of flow in the channel. When the device is in use, the downstream section is at a uniform temperature T_{H}, and the upstream section is at a temperature which is higher than T_{H}. Alternatively or additionally, each cooling zone associated with a portion of the channel comprises an upstream section and a downstream section relative to the direction of flow in the channel. When the device is in use, the downstream section is at a uniform temperature Tc, and the upstream section is at a temperature which is lower than Tc.

In some embodiments, the downstream section and the upstream section may be, in each heating zone and/or in cooling zone, insulated from each other via an insulation material placed between the downstream section and the upstream. When two or more upstream heating (or cooling) thermoelectric modules are present, each sub-section of the upstream section corresponding to one upstream heating (or cooling) thermoelectric module (i.e., the temperature of the sub-section is controlled by the corresponding thermoelectric module) may be insulated from another sub-section of the upstream section.

In some embodiments, when the device is in use, in each heating zone, the upstream section may be at a temperature which is at least 1°C higher than T_{H}, for example higher by from 1 °C to 30°C, preferably by from 5°C to 20°C. In each cooling zone, the upstream section may be at a temperature which is at least 1°C lower than Tc, for example, lower by from 1°C to 30°C, preferably by from 5°C to 20°C.

In some embodiments, the upstream section of each heating zone may be at a uniform temperature higher than T_{H}.

Alternatively or additionally, the upstream section of each cooling zone may be at a uniform temperature lower than Tc.

In some embodiments, the temperature of the upstream section of each heating zone may gradually or incrementally decrease in the upstream to downstream direction.

Alternatively or additionally, the temperature of the upstream section of each cooling zone may gradually or incrementally increase in the upstream to downstream direction.

The uniform temperature T_{H} may correspond to a temperature at which the denaturation of a nucleic acid occurs. For example, the uniform temperature T_{H} may be from 85°C to 100°C, preferably 90°C to 95°C.

The uniform temperature Tc may correspond to a temperature at which the annealing of primers and elongation of the primers by a polymerase occur. For example, the uniform temperature Tc may be from 50°C to 75°C, preferably 55°C to 65°C.

In some embodiments, the temperature regulation system may comprise a plurality of thermoelectric modules and preferably one or more heat spreading elements on one side of the thermoelectric modules.

Thermoelectric modules may also be designated as Peltier modules.

Below, the device is described as comprising thermoelectric modules; however, it is noted that other thermal components for regulating the temperature can be also used instead of, or in combination with thermoelectric modules.

For example, such thermal components may include heating resistors, or heat exchangers configured to exchange heat between the channel and a heat transfer fluid flowing through a heat transfer circuit.

Thermoelectric modules are preferred because a thermoelectric module may ensure better temperature homogeneity over a large surface area.

A heat spreading element may be any element which is thermally conductive and diffusive. For example, the heat spreading element may be a plate made of a metal, such as copper or aluminum, graphite, or a metal alloy such as brass (comprising copper and zinc). The heat spreading element is preferably a brass plate.

The heat spreading elements may be preferably attached on one side of the thermoelectric modules, for example using a thermally conductive adhesive.

In some embodiments, each heat spreading element may comprise one or more groove(s) or trench(es) on its surface for receiving the channel, preferably on the surface opposite the surface in contact with the thermoelectric module(s).

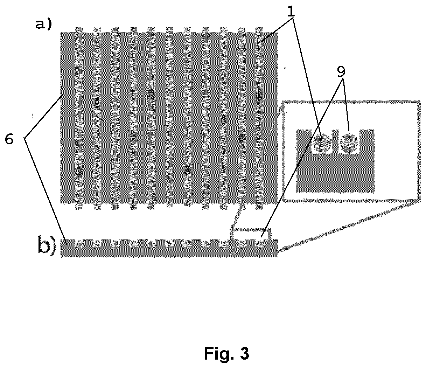

Each groove or trench may have a bottom wall and two side walls extending long an axis (which is preferably perpendicular to the central axis defined above), as shown in **Fig. 3****.**

The width of the bottom wall and the height of the side walls (perpendicular to the axis of extension of the groove or trench) may be from 1 mm to 5 mm, preferably from 1 mm to 3 mm.

The spacing between adjacent groove(s) or trench(es) may be from 0.01 mm to 5 mm, preferably from 0.1 mm to 1 mm.

The channel may be preferably attached in the groove(s) or trench(es), for example using a thermally conductive adhesive.

In such a configuration, the portions of the channel in contact with the heating and/or cooling zones may be partially in contact with the heating and/or cooling zones (and for example partially exposed to air, e.g. to the exterior environment), as explained above.

In other embodiments, each spreading element may be configured to embed the channel therein.

In such a configuration, the portions of the channel in contact with the heating and/or cooling zones may be entirely in contact with the heating and/or cooling zones, as explained above.

In preferred embodiments, the temperature regulation system may comprise a plurality of thermoelectric modules and one or more heat spreading elements on one side of the thermoelectric modules.

In some embodiments, the temperature regulation system may comprise a heating module comprising all heating zones and a cooling module comprising all cooling zones, as shown in **Fig. 1****.** In this case, the heating zones may be different sections of the same heating planar area in the heating module, and the cooling zones may be different sections of the same cooling planar area in the cooling module. The various sections in the heating planar area and cooling planar area may be physically distinct or may merely be conceptually defined in the heating planar area and cooling planar area.

The term *"planar"* refers to the overall shape of the area, but does not exclude the presence of three-dimensional structures on the surface, such as the grooves described above.

Such heating planar area and cooling planar area may be advantageously combined with the coiled channel, as shown in **Fig. 1****.**

In some embodiments, the heating planar area and the cooling planar area may face each other, and the coiled channel may be arranged between the heating planar area and the cooling planar area, with portions thereof in contact with the heating zones and the cooling zones as described above.

Such a configuration can result in a more compact device than using a flat channel configuration (such as serpentine-shaped or flat spiral-shaped channel).

A thermal insulator, such as polyethylene foam, may be placed between the heating planar area and the cooling planar area for thermally insulating one from the other.

In some embodiments, the temperature regulation system may comprise different thermoelectric modules for controlling the temperature of the upstream sections and downstream sections in the heating zones.

Preferably, these different thermoelectric modules are arranged in parallel, and extend in a direction perpendicular to the direction of flow in the channel.

This configuration allows, especially when the portions of the channel in contact with the heating zones are arranged in parallel (for example, when the channel is coiled, as shown in **Fig. 1**), one or more thermoelectric modules to control the temperature of the upstream sections in the heating zones over several portions of the channel (preferably over all portions of the channel in contact with heating zones), and another thermoelectric module to control the temperature of the downstream sections in the heating zones over several portions of the channel (preferably over all portions of the channel in contact with heating zones).

Likewise, especially when the portions of the channel in contact with the cooling zones are arranged in parallel (for example, when the channel is coiled, as shown in **Fig. 1**), the above configuration allows one or more thermoelectric modules to control the temperature of the upstream sections in the cooling zones over several portions of the channel (preferably over all portions of the channel in contact with cooling zones), and another thermoelectric module to control the temperature of the downstream sections in the cooling zones over several portions of the channel (preferably over all portions of the channel in contact with cooling zones).

In some embodiments, the temperature regulation system may comprise a single downstream heating thermoelectric module for controlling the temperature of the downstream sections of all heating zones, as shown in **Fig. 2****.**

In some embodiments, the temperature regulation system may comprise one or more upstream heating thermoelectric modules for controlling the temperature of the upstream sections of all heating zones, as shown in **Fig. 2****.**

For example, the temperature regulation system may comprise one upstream heating thermoelectric module for controlling the temperature of the upstream sections of all heating zones.

Alternatively, the temperature regulation system may comprise two or more, three or more, or four or more, or five or more upstream heating thermoelectric modules for controlling the temperature of the upstream sections of all heating zones.

In preferred embodiments, among the two or more upstream heating thermoelectric modules in the upstream sections, the most upstream heating thermoelectric module may be configured to control the temperature to the highest temperature, the second most upstream heating thermoelectric module may be configured to control the temperature to the second highest temperature, and so on (depending on the number of upstream heating thermoelectric modules), with the least upstream heating thermoelectric module being configured to control the temperature to the lowest temperature (and temperature closest to the uniform temperature T_{H} of the downstream sections).

It is to be noted that the temperatures of the above upstream sections are higher than the uniform temperature T_{H} of the downstream sections.

In some embodiments, the temperature regulation system may comprise different thermoelectric modules for controlling the temperature of the upstream sections and downstream sections in the cooling zones.

Preferably, these different thermoelectric modules are arranged in parallel, and extend in a direction perpendicular to the direction of flow in the channel.

One advantage of such a configuration is as explained above in relation to the arrangement of different thermoelectric modules in the heating zones.

In some embodiments, the temperature regulation system may comprise a single downstream cooling thermoelectric module for controlling the temperature of the downstream sections of all cooling zones, as shown in **Fig. 1****.**

In some embodiments, the temperature regulation system may comprise one or more upstream cooling thermoelectric modules for controlling the temperature of the upstream sections of all cooling zones, as shown in **Fig. 1****.**

For example, the temperature regulation system may comprise one upstream cooling thermoelectric module for controlling the temperature of the upstream sections of all heating zones.

Alternatively, the temperature regulation system may comprise two or more, three or more, or four or more, or five or more upstream cooling thermoelectric modules for controlling the temperature of the upstream sections of all heating zones.

In preferred embodiments, among the two or more upstream cooling thermoelectric modules in the upstream sections, the most upstream cooling thermoelectric module may be configured to control the temperature to the lowest temperature, the second most upstream cooling thermoelectric module may be configured to control the temperature to the second lowest temperature, and so on (depending on the number of upstream cooling thermoelectric modules), with the least upstream cooling thermoelectric module being configured to control the temperature to the highest temperature (and temperature closest to the uniform temperature Tc of the downstream sections).

It is to be noted that the temperatures of the above upstream sections are lower than the uniform temperature Tc of the downstream sections.

When the temperature regulation system comprises one or more thermoelectric modules in the upstream sections of heating zones and/or cooling zones, the phrase *"the upstream section is at a temperature which is higher (or lower) than T_{H} (or Tc)"* means that any temperature in the upstream sections is higher (or lower) than T_{H} (or Tc). In addition, the phrase *"the upstream section may be at a temperature which is higher (lower) than* T_{H} *(Tc) by* ...°C" means that the highest (or lowest) temperature in the upstream sections is higher (lower) than T_{H} (T_{C1}) by ... °C.

In some embodiments, the temperature regulation system may be configured to adjust a temperature profile of the upstream sections of the heating zones and/or a temperature profile of the upstream sections of the cooling zones as a function of the flow rate in the channel.

More specifically, when the temperature regulation system comprises a single upstream heating thermoelectric module in the upstream sections of the heating zones, the temperature of the upstream sections may be increased as the flow rate in the channel is increased. Alternatively or additionally, when the temperature regulation system comprises a single upstream cooling thermoelectric module in the upstream sections of the cooling zones, the temperature of the upstream sections may be decreased as the flow rate in the channel is increased.

More specifically, when the temperature regulation system comprises two or more upstream heating thermoelectric modules in the upstream sections of the heating zones, the temperature of at least one of the upstream heating thermoelectric modules (possibly of all upstream heating thermoelectric modules) may be increased as the flow rate in the channel is increased. Alternatively or additionally, when the temperature regulation system comprises two or more upstream cooling thermoelectric modules in the upstream sections of the cooling zones, the temperature of at least one of the upstream cooling thermoelectric modules (possibly of all upstream cooling thermoelectric modules) may be decreased as the flow rate in the channel is increased.

In some embodiments, the temperature regulation system may further comprise a temperature feedback control system connected to the thermoelectric modules (or other thermal components).

The temperature feedback control system may comprise one or more thermal sensors for measuring a temperature. The thermal sensors may communicate temperature data to a control module, and the control module may exchange data and instructions with the thermoelectric modules (or other thermal components). The thermoelectric modules (or other thermal components) may be controlled by the control module in order to reach or maintain over time one or more target temperatures based on the temperature data, based for example on a proportional-integral-derivative (PID) control loop. The control unit may also receive external input from a user. The control unit may be built in the device itself, or may be external to the device (for example this function may be carried out by a personal computer).

The thermal sensors may be positioned at respective locations suitable for measuring the temperature of upstream sections and downstream sections of heating zones and/or cooling zones. For example, the thermal sensors may be positioned in contact with the portions of the channel in contact with the heating zones and/or in contact with the portions in contact with the cooling zones, such as sandwiched between these portions and the respective heating zones or cooling zones.

When the temperature regulation system comprises one or more heat spreading elements (e.g., a brass plate), the thermal sensors may be embedded in the heat spreading elements, preferably in close proximity to the channel (for example at a distance of less than 5 mm, or less than 2 mm, or less than 1 mm from the channel).

As an example, the temperature feedback control system may comprise one thermal sensor embedded at the center of each heat spreading element (e.g., a brass plate). Alternatively, the temperature feedback control system may comprise a plurality of thermal sensors embedded in the heat spreading element (e.g., a brass plate). The thermal sensors may be spaced at an equal interval, preferably arranged symmetrically.

In some embodiments, the temperature regulation system may further comprise a heat dissipation system.

The heat dissipation system can result in improved heat convection and reduced thermal stress, thereby improving efficiency and safety of the temperature regulation system.

Any conventional heat dissipation system may be used, such as a heat sink, heat spreader, and/or a cooling fan, and/or a liquid cooling system, preferably a heat sink as shown in **Fig. 1** and **Fig. 2****.** As an example, the heat sink may comprises a plurality of protrusions or fins that increase the surface area for heat dissipation.

In a case where the temperature regulation system comprises a plurality of thermoelectric modules and one or more heat spreading elements one side of the thermoelectric modules, the heat dissipation system may be positioned on the other side of the thermoelectric modules. More preferably, the heat dissipation system may be attached on the other side of the thermoelectric modules, using a thermally conductive adhesive.

In some embodiments, in each heating zone, the ratio of the length of the upstream section to the length of the downstream section, along the direction of flow in the channel, is from 1:2 to 1:50, preferably from 1:3 to 1:20, more preferably from 1:4 to 1:10.

Alternatively or additionally, in each cooling zone, the ratio of the length of the upstream section to the length of the downstream section, along the direction of flow in the channel, is from 1:2 to 1:50, preferably from 1:3 to 1:20, more preferably from 1:4 to 1:10.

In some embodiments, the cooling zones may comprise first cooling zones and second cooling zones, wherein, when the device is in use:
- each first cooling zone comprises an upstream section and a downstream section relative to the direction of flow in the channel, the downstream section is at a uniform temperature T_{C1}, the upstream section is at a temperature which is lower than T_{C1}, and:
- each second cooling zone comprises an upstream section and a downstream section relative to the direction of flow in the channel, the downstream section is at a uniform temperature T_{C2}, the upstream section is at a temperature which is lower than T_{C2}.

The description above in relation to the cooling zones applies similarly to both the first and second cooling zones.

The terms *"first (cooling zone)"* and *"second (cooling zone)"* are simply used to differentiate between the two zones for ease of reference.

For example, among three different uniform temperatures T_{H}, T_{C1} and T_{C2}, T_{H} may be the highest temperature, T_{C1} may be the lowest temperature, and T_{C2} may be an intermediate temperature between T_{H} and T_{C1}. In this case, the uniform temperature T_{H} may correspond to a temperature for denaturation, as defined above. The uniform temperature T_{C1} may correspond to a temperature at which the annealing of primers can occur, for example, from 55°C to 65°C, preferably 55°C to 60°C. The uniform temperature T_{C2} may correspond to a temperature at which the elongation by a polymerase can occur, for example, from 55°C to 72°C, preferably 60°C to 65°C.

Alternatively, among the uniform temperatures T_{H}, T_{C1} and T_{C2}, T_{H} may be the highest temperature, T_{C2} may be the lowest temperature, and T_{C1} may be an intermediate temperature between T_{H} and T_{C2}. In this case, the uniform temperature T_{H} may correspond to a temperature for denaturation, as defined above. The uniform temperature T_{C2} may correspond to a temperature at which the annealing of primers can occur, for example, from 55°C to 65°C, preferably 55°C to 60°C. The uniform temperature T_{C1} may correspond to a temperature at which the elongation by a polymerase can occur, for example, from 55°C to 72°C, preferably 60°C to 65°C.

The placement of the first and second cooling zones relative to the heating zones in the temperature regulation systems may be appropriately adjusted depending on the uniform temperature of the first and second cooling zones. For example, when T_{C1} is higher than T_{C2}, each first cooling zone may be positioned downstream of the second cooling zone and upstream of the subsequent heating zone. Alternatively, when T_{C2} is higher than T_{C1}, each second cooling zone may be positioned downstream of the first cooling zone and upstream of the subsequent heating zones.

The channel comprising portions in contact with heating zones and cooling zones may comprise successive repeated patterns along the length thereof, wherein each pattern comprises (in the upstream to downstream direction) a heating zone, a first cooling zone and a second cooling zone; or a heating zone, a second cooling zone and a first cooling zone.

When first cooling zones and second cooling zones are present, then the temperature regulation system may comprise a first cooling planar area comprising all first cooling zones and a second cooling planar area comprising all second cooling zones.

Alternatively, the cooling zones do not comprise first and second cooling zones and the downstream sections of all cooling zones are at a same uniform temperature Tc when the device is in use.

The configuration wherein the cooling zones do not comprise first and second cooling zones is suitable for implementing two-step PCR, as described above. The configuration wherein the cooling zones comprise first and second cooling zones is suitable for implementing three-step PCR, as described above.

In some embodiments, the device of the invention may further comprise a measurement system for quantifying the amplified nucleic acids. Such a measurement system is already well known in the domain, for example a fluorescence dye-based or fluorescence probe-based system. In this case, the device may be configured to perform quantitative amplification, for example qPCR, preferably by quantifying the amplified nucleic acids in individual droplets.

The measurement may be carried out on the droplets as they flow through portions of the channel which are in contact/are not in contact with heating zones and cooling zones.

The measurement system may comprise a camera, a light source, and an optical system.

The camera may be a high-sensitivity camera capable of detecting and measuring fluorescence, such as a CCD (charge-coupled device) camera, a CMOS (complementary metal-oxide-semiconductor) camera or a photodiode.

The light source may be any light source capable of exciting fluorescent dyes or probes. For example, the light source may be an LED light, a argon-laser, or a halogen lamp.

The optical system may comprise, for example, optical filters such as excitation and emission filters, lenses and mirrors.

In some embodiments, the measurement system may be positioned near the channel, preferably near the portions of the channel which are in contact with heating zones and cooling zones. In this case, the temperature regulation system (for example, thermoelectric module(s) and/or heat spreading element(s)) may be transparent, or may comprise a window for taking measurements.

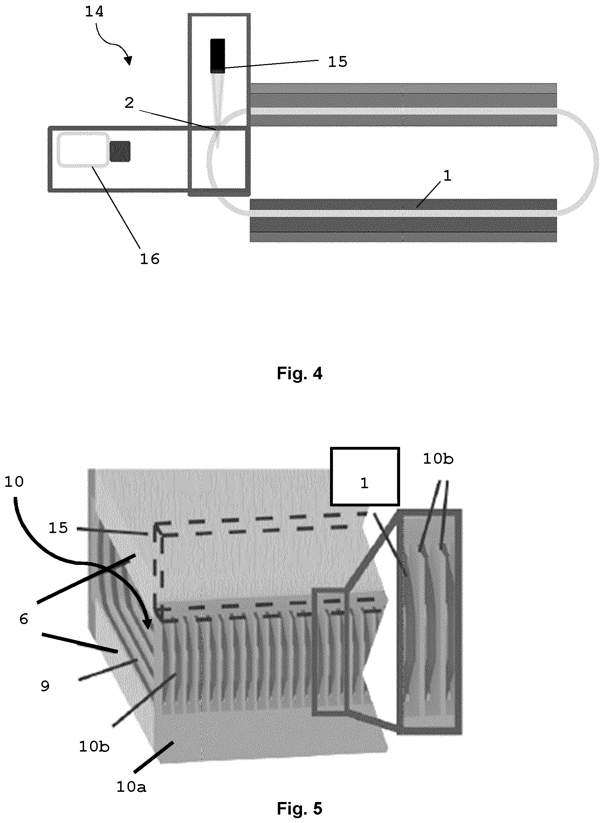

In preferred embodiments, the measurement system may be positioned near the channel, preferably near the portions of the channel which are not in contact with heating zones and cooling zones, as shown in **Fig. 4****.**

When the channel is coiled, the measurement system may be configured to simultaneously carry out measurements in multiple coils of the channel. In particular, the field of view of the camera may encompass multiple adjacent loops (coils), for example 2 to 100 loops (coils), or 5 to 75 loops (coils), or 20 to 60 loops (coils).

In such a configuration, the device may further comprises a comb-like structure for securing the channel of the device, as shown in **Fig. 5****.**

This arrangement helps stabilize the position of the channel and prevent light interference between the loops of the channel (in portions not in contact with heating zones and cooling zones) during simultaneous measurement on multiple loops.

The comb-like structure may comprise a base with parallel walls extending from the base.

The comb-like structure may further comprise slots between the parallel walls. The slots are configured to receive respective portions of the channel.

The spacing between these walls (the distance between adjacent slots) may thus correspond to the spacing of the loops of the coiled channel, for example from 10 µm to 10 mm, preferably from 100 µm to 1 mm

The base of the comb-like structure may be positioned on the opposite side of the channel from the light source, as shown in **Fig. 5****,** ensuring that the base does not interfere with the light emitted by the light source.

The device may further comprise a droplet generator for generating droplets flowing through the channel as described above. The droplet generator may be integrated in the droplet manipulation mechanism, as will be described in more detail below.

Any conventional droplet generator may be used, for example, a flow-focusing droplet generator, a co-flowing droplet generator or a T-junction droplet generator. These generators are very well known in the domain.

As another example, the droplet generator may be configured to generate droplets by pipetting. The droplet generator by pipetting allows for the analysis of multiple samples and nucleic acids with no risk of contamination.

Such a droplet generator by pipetting may comprise one or more channels, and one or more fluid reservoirs (each fluid reservoir may comprise two or more containers).

The droplet generator may directly feed droplets within the main fluid to the channel comprising portions in contact with heating zones and cooling zones of the temperature regulation system, or may first feed droplets within the main fluid to an intermediate module upstream of this channel, such as an RT module (which will be explained below).

The fluid reservoir may preferably comprise at least one container for storing main fluid, as explained above, and at least one container for storing an aqueous solution, as explained above.

The fluid reservoir may be, for example, a well plate, such as a microtiter plate or a microwell plate. In this case, each container may be a well of the well plate.

The droplet generator by pipetting may be configured to generate droplets by alternatingly pipetting the aqueous solution stored in one container of the fluid reservoir (e.g., a well of a microtiter plate) and the main fluid stored in another container of the fluid reservoir (e.g., another well of the microtiter plate).

Such a pipetting operation may be performed manually or by using an arm robot.

In some embodiments, the fluid reservoir may comprise three or more containers, one container for storing main fluid and two or more containers for storing two or more different aqueous solutions.

For example, in the case of a microtiter plate, the microtiter plate may comprise three wells, one well for storing the main fluid, one well for storing an aqueous solution containing a nucleic acid to be amplified (or a nucleic acid to be reverse-transcribed and then amplified, which will be described later), and one well for storing an aqueous solution containing reagents, such as reagents for amplifying the nucleic acid (and optionally RT reagents).

A plurality of containers (a plurality of wells) may contain different nucleic acids to be amplified. Additionally, or alternatively, a plurality of containers (a plurality of wells) may contain different aqueous solutions containing reagents (for example, containing different primers).

Especially when the droplet manipulation mechanism comprises one or more syringe pump(s) and valve(s), the above described droplet generator by pipetting makes it possible, in combination with the valves, to achieve a throughput which is not limited by the droplet generation rate. More specifically, multiple individual droplets can be generated sequentially while these droplets flow continuously in close proximity, allowing them to merge.

For example, in the above example of the microtiter plate comprising three or more wells, with two wells for two different aqueous solutions, a droplet of a first aqueous solution containing a nucleic acid and a droplet of a second aqueous solution containing reagents can be generated individually, these droplets may be caused to merge, thereby forming a droplet containing a nucleic acid and reagents which may then flow to the channel comprising portions in contact with heating zones and cooling zones of the thermal regulation system.

In some embodiments, the device may further comprise a reverse transcription (RT) module. Any conventional RT module, preferably microfluidic RT module, may be used.

Thus, the device of the invention may be also configured to perform reverse transcription of ribonucleic acids (RNAs) to complementary deoxyribonucleic acids (cDNAs) and amplification of the cDNAs, for example RT-PCR.

In preferred embodiments, the device may comprise a measurement system as described above and an RT module as described above, and thus may be configured to perform RT-qPCR.

The RT module may be placed upstream of the thermal regulation system of the device.

The RT module may comprise an RT channel for flowing a droplet containing a nucleic acid and RT reagents and reagents for amplifying the nucleic acid, and a thermal system.

In this case, the nucleic acid may be a ribonucleic acid (RNA).

The inlet of the RT channel may be connected to (adjoin) the outlet of the droplet generator.

The outlet of the RT channel may be connected to (adjoin) the channel comprising portions in contact with heating zones and cooling zones of the thermal regulation system.

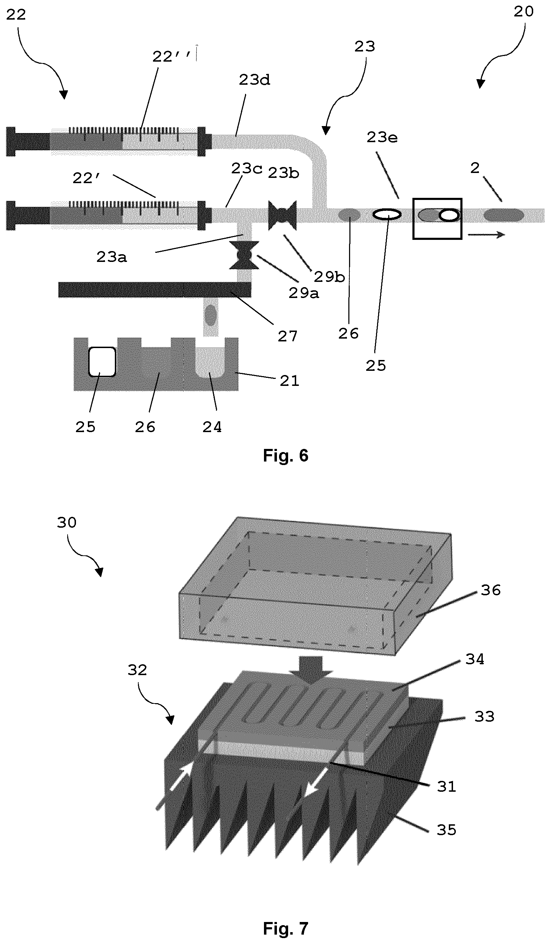

The RT channel may be curved, for example serpentine-shaped, as shown in **Fig. 7****.**

The thermal system may comprise a thermoelectric module and a heat spreading element.

As explained above in relation with the thermoelectric modules of the temperature regulation system of the device, the thermal system may comprise another thermal component, such as a heating resistor or a heat exchanger, instead of, or in combination with the thermoelectric module.

The thermal component may be configured to control the temperature of the thermal system, which is in contact with the RT channel.

Preferably, the area of the thermal system (preferably the area of the heat spreading element) in contact with the RT channel, has a uniform temperature, in use. This uniform temperature may correspond to a temperature at which the reverse transcription of a ribonucleic acid occurs.

For example, the uniform temperature may be from 35°C to 60°C, preferably from 50°C to 55°C.

The thermal system may further comprise a heat dissipation system.

The heat dissipation system may be as described above in relation with the heat dissipation system of the temperature regulation system of the device.

The RT module may further comprise a lid for covering the thermal system in order to guarantee thermal insulation from the external environment.

The lid can be made from a variety of conventional plastics, such as ABS (acrylonitrile butadiene styrene).

**Fig. 1** shows an embodiment of the device of the invention.

The device comprises a channel **1,** and a temperature regulation system 3. A droplet manipulation mechanism is not shown.

The channel **1** comprises portions in contact with heating zones of the temperature regulation system **3** alternating with portions in contact with cooling zones of the temperature regulation system **3.**

Each heating zone and/or each cooling **5** zone comprises an upstream section **51** and a downstream section **52** (indicated by two rectangles) relative to the direction of flow in the channel (as indicated by an arrow in the channel in which droplets **2** flow).

The channel **1** is coiled, *i.e*. comprises multiple coils. Each coil comprises one portion in contact with a heating zone, and one portion in contact with a cooling zone.

Each cooling zone or heating zone is a zone of the temperature regulation system which is in contact with the channel. The various cooling zones (respectively heating zones) may be of unitary construction, for example they may belong to a respective (preferably one-piece) heat spreading element. The various cooling zones (respectively heating zones) may be formed as individual grooves on the respective heat spreading element. Alternatively, the various cooling zones (respectively heating zones) may simply be different areas on a flat or curved surface of the respective heat spreading element.

In the illustrated embodiment, the temperature regulation system comprises a heating module **8** and a cooling module **7,** each of which may comprise two thermoelectric modules **7a, 7b** and respective heat spreading elements **6** (for example brass plates), each on one side of each thermoelectric module **7, 8.**

In this embodiment, the heating module comprises a heating planar area comprising all heating zones and the cooling module comprises a cooling planar area comprising all cooling zones.

It is to be noted that **Fig. 1** illustrates a part of the entire device for ease of reference; however, the coiled channel is present on the other side of the device (*i*.*e*., the side facing away from the viewer) in the same way as shown on the near side (*i*.*e*., side closer to the viewer), as shown in **Fig. 4****.**

In this embodiment, the temperature regulation system **3** comprises different thermoelectric modules for controlling the temperature of the upstream sections and downstream sections in the heating zones and/or cooling zones, more specifically one upstream cooling thermoelectric module **7a** (respectively upstream heating thermoelectric module, not shown) for controlling the temperature of the upstream sections of all cooling zones (respectively heating zones) and one downstream cooling thermoelectric module **7b** (respectively downstream heating thermoelectric module, not shown) for controlling the temperature of the downstream sections of all cooling zones (respectively heating zones).

These different thermoelectric modules **7a, 7b** are arranged in parallel, perpendicular to the direction of flow in the channel. They may extend parallel to the central axis of the coiled channel as described above. In the enlarged portion of **Fig. 1****,** the thermoelectric modules **7a, 7b** are shown in a cross-section perpendicular to their main direction of extension.

The temperature regulation system may further comprise a temperature feedback control system comprising a control module **11** and one or more thermal sensors **12,** preferably embedded in the respective heat spreading elements \**6** as described above. The temperature regulation system may further comprise a heat sink **13** (heat dissipation system) arranged on the opposite side of the thermoelectric modules **7, 8.**

As shown in **Fig. 3(a)** and **Fig. 3(b)****,** each heat spreading element **6** (*e*.*g*., brass plate) may comprise grooves **9** on its surface for receiving the channel **1,** on the surface opposite the surface in contact with the thermoelectric modules (not shown). In this case, each heating zone or cooling zone may correspond to a respective groove.

**Fig. 2** shows a partial view of the temperature regulation system according another embodiment of the device of the invention. The device is the same as the device shown in **Fig. 1****,** except that the heating (or cooling) module **8** of the temperature regulation system **3** comprises three upstream heating (or cooling) thermoelectric modules **8a, 8a', 8a"** for controlling the temperature of the upstream sections **41** of all heating (or cooling) zones **4,** and one downstream heating (or cooling) thermoelectric modules **8b** for controlling the temperature of the downstream sections **42** of all heating (or cooling) zones **4.**

**Fig. 4** shows another embodiment of the device of the invention. The device may be the same as the device in relation to **Fig. 1****,** except that the device further comprises a measurement system **14** for quantifying the amplified nucleic acids.

The measurement system **14** may comprise an LED light **15** (light source) and a CMOS camera **16.** The LED light **15** may be positioned such that it emits the light on droplets **2** flowing through portions of the channel which are not in contact with heating zones and cooling zones. The CMOS camera **16 may** be positioned such that the CMOS camera **16** detects the fluorescence emitted by the amplified nucleic acids contained in the droplet **2.** The optical system (not shown) may be positioned between the LED light **15** and the channel **1.**

As shown in **Fig. 5****,** the device may further comprises a comb-like structure **10** for securing the channel of the device. The device may be the same as the one described in relation to **Fig. 1****,** except that the device further comprises a measurement system of **Fig. 4****.**

The comb-like structure **10** may comprise a base **10a** with parallel walls **10b** extending from the base.

Thus, the comb-like structure **10** stabilizes the position of the coiled channel **1** during simultaneous measurements in multiple loops and prevent light interference between the loops of the channel (in portions not in contact with heating zones and cooling zones) during the measurement process.

The light source **15** of the measurement system may be positioned adjacent to the comb-like structure, for example, at a position indicated by the dotted-line rectangle of **Fig. 5****.**

**Fig. 6** shows one embodiment of a droplet manipulation mechanism **20** which is also a droplet generator.

The droplet manipulation mechanism **20** may comprise a fluid reservoir (microtiter plate **21**), a first syringe pump **22'** and a second syringe pump **22".**

The first syringe pump **22'** and second syringe pump **22"** may be partially filled with main fluid as described above. Instead of syringe pumps, pressure control devices coupled with a reservoir of main fluid may also be used.

The microtiter plate **21** may contain multiple wells containing various fluids. One or more wells may contain main fluid **24,** and one or more wells may contain aqueous solutions **25, 26** as will be further elaborated below.

The droplet manipulation mechanism **20** may also comprise a droplet merging channel **23e** as well as multiple channels **23a, 23b, 23c, 23d** for feeding main fluid and aqueous solutions to the droplet merging channel **23e.** In the illustrated configuration, these may include a pipetting channel **23a,** a first channel **23c,** a second channel **23d** and a connection channel **23b.**

A first valve **29a** (such as a pinch valve) may be present on the pipetting channel **23a** and a second valve **29b** (such as a pinch valve) may be present on the connection channel **23b.**

The first channel **23c** may be connected to an outlet of the first syringe pump **22'.**

The second channel **23d** may be connected to an outlet of the second syringe pump **22'.**

The pipetting channel **29a** may be connected to the fluid reservoir **21.**

The connection channel **23b** may have one end connected to both the first channel **23c** and the pipetting channel **23a** (as a T junction); and another end connected to both the second channel **23d** and the droplet merging channel **23e** (as a T junction).

An arm robot **27** may be coupled with the pipetting channel **23a** so as to displace an open end of the pipetting channel **23a,** for example according to a X-Y plane.

This makes it possible for the open end of the pipetting channel **23a** to be successively plunged into various wells of the microtiter plate **21.** When the second valve **29b** is closed and the first valve **29a** is open, pipetting may take place by actuating the first syringe pump **22'.** By way of example, a droplet of a first aqueous solution **25** and/or a droplet of a second aqueous solution **26** may be formed by moving the open end of the pipetting channel **23a** into different wells of the microtiter plate **21.**

For example the first aqueous solution **25** may contain a nucleic acid to be amplified and the second aqueous solution **26** may contain reagents as described above.

Main fluid **24** contained in another well of the microtiter plate **21** may also be pipetted between each pipetting of aqueous solution. The thus formed droplets may flow through the pipetting channel **23a** and into the first channel **23c.**

After this pipetting phase, the first valve **29a** may be closed and the second valve **29b** may be opened, and the first syringe pump **22'** may be actuated so as to displace the main fluid in the opposite direction (*i*.*e*. so as to expel the main fluid from the first syringe pump **22**'). As a result, the droplets may be displaced from the first channel **23a** through the connection channel **23b** and then into the droplet merging channel **23e.** This may be referred to as a droplet pushing phase.

In some variations, only one droplet is pipetted and pushed at each sequence of pipetting and pushing. In other variations, two or more, preferably only two, droplets are pipetted and then are simultaneously pushed at each such sequence.

Main fluid may be fed from the second syringe pump **22"** to the droplet merging channel **23e,** directly *via* the second channel **23d.** This feeding may take place separately from the pipetting and droplet pushing phases. Alternatively, it may take place continuously, simultaneously with the pipetting and droplet pushing phases, in order to ensure a continuous flow of main fluid to the droplet merging channel **23e** and downstream.

By adjusting the flow rate of main fluid in the droplet merging channel **23e,** and/or by providing a constriction or other known merging inducing feature, droplets (for example one droplet of first aqueous solution **25** and one droplet of second aqueous solution **26**) may merge within the droplet merging channel **23e.**

The droplet merging channel **23e** may have an inlet and an outlet. The inlet may be connected to the second channel **23d** and the connection channel **23b** as described above.

The outlet of the droplet merging channel **23e** may be connected to the inlet of the channel comprising portions in contact with heating zones and cooling zones of the temperature regulation system, or may be connected to the inlet of an RT channel (in which case the outlet of the RT channel may be connected to the inlet of the channel comprising portions in contact with heating zones and cooling zones of the temperature regulation system). Therefore, after merging, the obtained droplet **2** may flow through (optionally, the RT channel, and then) the channel having portions in contact with heating zones and cooling zones.

Although merging of two droplets was described above, it is also possible to generate a droplet by merging three or more droplets before feeding the merged droplet to (optionally, the RT channel, and then) the channel having portions in contact with heating zones and cooling zones.

Conversely, it is also possible to generate one or more droplets without any merging. In this case, one or more aqueous solutions containing all necessary components for the PCR reaction (and optionally RT reaction) may be placed in respective wells of the microtiter plate **21.** Individual droplets may be pipetted and pushed as described above, and fed without merging to (optionally, the RT channel, and then) the channel having portions in contact with heating zones and cooling zones.

**Fig. 7** shows an embodiment of the RT module which may be used in the device of the invention.

The RT module **30** may comprise an RT channel **31** for flowing a droplet containing a nucleic acid and RT reagents and reagents for amplifying the nucleic acid, and a thermal system **32.**

The direction of the flow in the channel is indicated by two white arrows.

The inlet of the RT channel **31** of the RT module may be connected to the outlet of the droplet merging channel **23e** as described above.

The outlet of the RT channel **31** of the RT module may be connected to the inlet of the channel comprising portions in contact with heating zones and cooling zones of the temperature regulation system.

In this example, the thermal system **32** comprises a thermoelectric module **33,** a heat spreading element **34** (for example brass plate), a heat dissipation system **35** (for example, a heat sink), and a lid **36.** The heat spreading element **34** may be arranged on one side of the thermoelectric module **33** and the heat dissipation system **35** may be arranged on the opposite side of the thermoelectric module **33.** The RT channel **31** may disposed on the heat spreading element **34,** for example in one or more grooves on the surface of the heat spreading element **34** opposite the surface contacting the thermoelectric module 33. The lid **36** may cover the RT channel **31,** heat spreading element **34** and/or thermoelectric module **33.**

### Method for nucleic acid amplification

The method of amplifying a nucleic acid may comprise:
- providing at least one droplet containing a nucleic acid and reagents for amplifying said nucleic acid,
- flowing the droplet through a channel and alternately heating and cooling the droplet as the droplet flows through the channel, wherein:
- each step of heating the droplet comprises heating the droplet by flowing the droplet through a portion of the channel in contact with a heating zone comprising an upstream section which is at least one temperature which is higher than a uniform temperature T_{H} and a downstream section which is at the uniform temperature T_{H}; and/or each step of cooling the droplet comprises cooling the droplet by flowing the droplet through a portion of the channel in contact with a cooling zone comprising an upstream section which is at least one temperature which is lower than a uniform temperature Tc and a downstream section which is at a uniform temperature Tc.

The method for nucleic acid amplification may be performed using the device as described above.

What is described above in relation to the device for amplifying a nucleic acid applies similarly to the method for amplifying a nucleic acid.

The method of amplifying a nucleic acid using the above device comprises:
- providing at least one droplet containing a nucleic acid and reagents for amplifying said nucleic acid,
- flowing the droplet along the channel and alternatively heating and cooling the droplet as the droplet flows through portions in contact with heating zones and portions in contact with cooling zones.

The step of providing at least one droplet containing a nucleic acid and reagents for amplifying may be performed following a conventional droplet generation protocol, such as by using a flow-focusing droplet generator or a T-junction droplet generator.

The step of providing at least one droplet containing a nucleic acid and reagents for amplifying may comprise providing the droplet by pipetting, for example, using a droplet generator by pipetting, as explained above.

The droplet may be formed by merging two or more initial droplets, as described above.

The nucleic acid may be deoxyribonucleic acid (DNA) or ribonucleic acid (RNA), preferably DNA.

The reagents for amplifying the nucleic acid may comprise a DNA polymerase, and the nucleic acid to be amplified (target nucleic acid), primers, deoxynucleotide triphosphates (dNTPs). All reagents may be formulated in a buffer solution.

Preferably, multiple droplets are generated so that they simultaneously travel along the channel, as a train of successive droplets.

When successive droplets travel along the channel, they may have the same initial content or preferably different contents, so that different PCR reactions are implemented in the various droplets. For example, the droplets may contain different nucleic acids to be amplified (from different samples) and/or may contain different primers.

Any conventional thermostable DNA polymerase may be used, for example, Taq polymerase, Pfu polymerase, or other thermostable DNA polymerases.

The reagents may further comprise one or more additives for example, magnesium chloride (MgCl₂) for stabilizing the activity of DNA polymerase, and/or another stabilizer such as DMSO (dimethyl sulfoxide), and/or BSA (bovine serum albumin).

Especially when quantitative amplification (i.e., qPCR) is performed, the reagent may further comprise a fluorescent dye or probe, for example, SYBR Green, TaqMan probes or Molecular Beacons.

In some embodiments, the method may further comprise a step of reverse-transcribing (RT) the nucleic acid in a droplet after the step of providing at least one droplet containing a nucleic acid and reagents for amplifying the nucleic acid, and before the step of alternately heating and cooling the droplet.

In this case, the nucleic acid in the provided droplet is a ribonucleic acid (RNA), and the reagents for amplifying the nucleic acid may further comprise RT reagents.

The RT reagents may comprise a reverse transcriptase, primers, dNTPs, and one or more additives.

In some embodiments, the nucleic acid contained in the droplet may be a transcribed nucleic acid. In this case, the nucleic acid is a DNA transcribed from an RNA molecule, more specifically cDNA.

In such embodiments, the method may further comprise a preliminary step of reverse-transcribing a nucleic acid, and providing a droplet containing the transcribed nucleic acid and reagents for amplifying the nucleic acid.

The reverse transcription reaction may be performed using a conventional protocol, for example, by preparing a reaction mix comprising the nucleic acid (ribonucleic acid) to be transcribed, and RT reagents.

The step of flowing the droplet along the channel may be performed using any suitable conventional flow regulator as described above.

Each droplet flowing through the channel may have a volume from 0,1 nL to 10 µL, preferably from 5 nL to 1 µL, more preferably from 50 nL to 500 nL. Larger or smaller scale implementations are however also possible.

The flow rate of the main fluid may be from 0,1 nL/s to 10 µL/s, preferably from 50 nL/s to 500 nL/s. Higher or lower flow rates can be implemented to adapt the scale of the system and the targeted application.

The step of alternatively heating and cooling the droplet may be performed, as explained above, using a temperature regulation system comprising a plurality of thermoelectric modules and one or more heat spreading elements.

During the steps of heating the droplet as the droplet flows through portions in contact with heating zones, nucleic acid denaturation may occur in the droplet.

During the steps of cooling the droplet as the droplet flows through portions in contact with cooling zones, primer annealing and primer elongation by a polymerase may occur in the droplet.

In the method of the invention, the alternation of the step of heating the droplet as the droplet flows through portions in contact with heating zones and the step of cooling the droplet as the droplet flows through portions in contact with cooling zones may be repeated for 10 to 100 cycles, preferably 15 to 75 cycles, more preferably 20 to 50 cycles.

In some embodiments, as the droplet flows through a portion in contact with a heating zone, the temperature of the droplet varies and then remains constant (as defined above); and/or as the droplet flows through a portion in contact with a cooling zone, the temperature of the droplet varies and then remains constant.

In some embodiments, the temperature of the droplet gradually or incrementally increases in the upstream to downstream direction as the droplet flows through a portion in contact with a heating zone before becoming constant; and/or the temperature of the droplet gradually or incrementally decreases before becoming constant in the upstream to downstream direction as the droplet flows through a portion in contact with a cooling zone. In other, alternative embodiments, more complex variations are possible. For example, the temperature of the droplet may increase above the target temperature, then decrease, then become constant, in the upstream to downstream direction, as the droplet flows through a portion in contact with a heating zone; and the temperature of the droplet may decrease below the target temperature, then increase, then become constant, in the upstream to downstream direction, as the droplet flows through a portion in contact with a cooling zone.

In some embodiments, as the droplet flows through a portion in contact with a heating zone, the ratio of the duration for which the temperature of the droplet varies and is not constant (thermalization time) to the total duration for which the droplet flows through the portion in contact with a heating zone is from 1 to 60%, preferably from 2 to 50%, more preferably from 5 to 40%, and most preferably from 10 to 30%; and/or as the droplet flows through a portion in contact with a cooling zone, the ratio of the duration for which the temperature of the droplet varies and is not constant (thermalization time) to the total duration for which the droplet flows through the portion in contact with a cooling zone is from 1 to 60%, preferably from 2 to 50%, more preferably from 5 to 40%, and most preferably from 10 to 30%.

Within the above ranges, the steady state at the uniform temperature, which is necessary for efficient amplification reactions, can be ensured while the thermalization time for reaching the uniform temperature can be reduced, as explained above.

### EXAMPLES

A device of the invention was partially simulated using a computational fluid dynamics (CFD) model. Specifically, the thermalization time of a cooling zone of the temperature regulation system of the device was simulated.

The temperature regulation system was composed of a brass plate and a heat source (thermoelectric module) attached at the base of the brass plate.

The channel was composed of a PTFE tube embedded in the brass plate.

The dimension of each component was as follows:
- PTFE tube: diameter of 0.3 mm, total length of 40 mm and wall thickness of 0.15 mm.
- Brass plate: thickness of 3 mm.

In the above device, a CFD model was used to simulate a flow of a water droplet (200 nL) in oil (fluorinated carbon, FC-40) within the channel, under the following conditions:
- Temperature of the flow (for both the water droplet and oil) at the channel inlet: 95°C.
- Temperature of the upstream section of the cooling zone: 40 to 50°C.
- Temperature of the downstream section of the cooling zone: 60°C (corresponding to the uniform temperature T_{H} of the invention).

In the cooling zone, the upstream section and the downstream section were insulated from each other.

The flow in the channel was modeled as laminar, as indicated by the Reynolds number (Re) of < 1 (indicating low turbulence).

The fluid flow inside the channel was simulated using the Volume of Fluid (VOF) model, which accounts for surface tension as the primary interaction between the two phases.

The time required for cooling the water droplet from 95°C to a temperature of 60°C+/-0.2°C, was simulated.

The results are shown below.

**Table 1**

| | | | | |
|---|---|---|---|---|
| | Comparative Example 1 | Example 1 | Example 2 | Example 3 |
| Total duration on brass plate (s) | 4.7 | 4.7 | 4.7 | 4.7 |
| Thermalization time (s) | 2.7 | 1.8 | 1.3 | 2.2 |
| Thermalization/duration on plate | 57.45% | 38.30% | 27.66% | 46.81% |
| Time at 60+/-0.2 °C (s) | 2 | 2.9 | 3.4 | 2.5 |

In Comparative Example 1, the temperatures of the upstream and downstream sections were 60°C.

In Example 1, the temperature of the upstream section was 40°C and the temperature of the downstream section (T_{H}) was 60°C.

In Example 2, the temperature of the upstream section was 45°C and the temperature of the downstream section (T_{H}) was 60°C.

In Example 3, the temperature of the upstream section was 50°C and the temperature of the downstream section (T_{H}) was 60°C.

The *"total duration on brass plate"* (in seconds) indicates that the total time that the water droplet spent on the brass plate while flowing through the channel embedded in the brass plate.

The *"thermalization time"* (in seconds) indicates the time required for cooling the water droplet from 95°C to a temperature of 60°C+/-0.2°C.

The *"thermalization*/*duration on plate"* (in %) indicates the percentage of thermalization time relative to the total time that the water droplet spent on the brass plate.

The *"time at 60+*/*-0.2 °C"* (in seconds) indicates the time that the water droplet spent at 60+/-0.2 °C on the brass plate.

The simulated results shown in Table 1 indicate that Examples 1 to 3 corresponding to the invention require a shorter time to reach the temperature of 60°C in the water droplet, resulting in reduced thermalization time, compared to the Comparative Example.

This indicates that the water droplet spent more time at the temperature of 60°C (see *"Thermalization*/*duration on plate* (%)" and *"Time at 60+*/*-0.2 °C (s)").*

In the context of PCR, the fluid inlet temperature of 95°C may correspond to the temperature of the denaturation step, while the fluid temperature of 60°C may correspond to the temperature of the annealing-elongation step.

Thus, Examples 1 to 3 can provide less time for the temperature transition from one PCR step to the subsequent PCR step, resulting in an efficient nucleic acid amplification.

## Claims

1. A device for nucleic acid amplification, comprising:
- a channel;
- a temperature regulation system;
- a droplet manipulation mechanism configured to flow a droplet along the channel;
wherein:
- the channel comprises portions in contact with heating zones of the temperature regulation system alternating with portions in contact with cooling zones of the temperature regulation system, the heating zones being configured to be at a higher temperature than the cooling zones,
and wherein:
- each heating zone comprises an upstream section and a downstream section relative to the direction of flow in the channel,
- the downstream section is configured to be at a uniform temperature T_{H},
- the upstream section is configured to be at a temperature which is higher than T_{H};
and/or:
- each cooling zone comprises an upstream section and a downstream section relative to the direction of flow in the channel,
- the downstream section is configured to be at a uniform temperature Tc,
- the upstream section is configured to be at a temperature which is lower than Tc.

2. The device of claim 1, wherein:
- the upstream section of each heating zone is configured to be at a uniform temperature higher than T_{H}; and/or
- the downstream section of each cooling zone is configured to be at a uniform temperature lower than Tc.

3. The device of claim 1 or 2, wherein:
- the upstream section of each heating zone is configured so that the temperature in this upstream section gradually or incrementally decreases in the upstream to downstream direction; and/or
- the upstream section of each cooling zone is configured so that the temperature in this upstream section gradually or incrementally increases in the upstream to downstream direction.

4. The device of any one of claims 1 to 3, wherein the channel is coiled.

5. The device of any one of claims 1 to 4, wherein the temperature regulation system comprises a plurality of thermoelectric modules and one or more heat spreading elements on one side of the thermoelectric modules.

6. The device of any one of claims 1 to 5, wherein the temperature regulation system comprises a heating module comprising all heating zones and a cooling module comprising all cooling zones, wherein the heating zones are preferably arranged along a same planar area of the heating module and the cooling zones are preferably arranged along a same planar area of the cooling module.

7. The device of any one of claims 1 to 6, wherein the temperature regulation system comprises:
- different thermoelectric modules for controlling the temperature of the upstream sections and downstream sections in the heating zones, wherein, preferably, these different thermoelectric modules are arranged in parallel, and extend in a direction perpendicular to the direction of flow in the channel; and/or
- different thermoelectric modules for controlling the temperature of the upstream sections and downstream sections in the cooling zones, wherein, preferably, these different thermoelectric modules are arranged in parallel, and extend in a direction perpendicular to the direction of flow in the channel.

8. The device of any one of claims 1 to 7, wherein the temperature regulation system comprises a single downstream heating thermoelectric module for controlling the temperature of the downstream sections of all heating zones; and/or a single downstream cooling thermoelectric module for controlling the temperature of the downstream sections of all cooling zones.

9. The device of any one of claims 1 to 8, wherein the temperature regulation system comprises one or more upstream heating thermoelectric modules for controlling the temperature of the upstream sections of all heating zones; and/or one or more upstream cooling thermoelectric modules for controlling the temperature of the upstream sections of all cooling zones.

10. The device of any one of claims 1 to 9, wherein:
- in each heating zone, the ratio of the length of the upstream section to the length of the downstream section, along the direction of flow in the channel, is from 1:2 to 1:50, preferably from 1:3 to 1:20, more preferably from 1:4 to 1:10; and/or
- in each cooling zone, the ratio of the length of the upstream section to the length of the downstream section, along the direction of flow in the channel, is from 1:2 to 1:50, preferably from 1:3 to 1:20, more preferably from 1:4 to 1:10.

11. The device of any one of claims 1 to 10, wherein the temperature regulation system is configured to adjust a temperature profile of the upstream sections of the heating zones and/or a temperature profile of the upstream sections of the cooling zones as a function of the flow rate in the channel.

12. A method of amplifying a nucleic acid using the device for nucleic acid amplification of any one of claims 1 to 11, comprising:
- providing at least one droplet containing a nucleic acid and reagents for amplifying said nucleic acid, and
- flowing the droplet along the channel and alternately heating and cooling the droplet as the droplet flows through portions in contact with heating zones and portions in contact with cooling zones.

13. The method of claim 12, wherein:
- as the droplet flows through a portion in contact with a heating zone, the temperature of the droplet varies and then remains constant; and/or
- as the droplet flows through a portion in contact with a cooling zone, the temperature of the droplet varies and then remains constant.

14. The method of claim 13, wherein:
- the temperature of the droplet gradually or incrementally increases in the upstream to downstream direction and then remains constant as the droplet flows through a portion in contact with a heating zone; and/or
- the temperature of the droplet gradually or incrementally decreases in the upstream to downstream direction and then remains constant as the droplet flows through a portion in contact with a cooling zone.

15. The method of any one of claims 13 to 14, wherein:
- as the droplet flows through a portion in contact with a heating zone, the ratio of the duration for which the temperature of the droplet varies and is not constant to the total duration for which the droplet flows through the portion in contact with a heating zone is from 1 to 60%, preferably from 2 to 50%, more preferably from 5 to 40%, and most preferably from 10 to 30%; and/or
- as the droplet flows through a portion in contact with a cooling zone, the ratio of the duration for which the temperature of the droplet varies and is not constant to the total duration for which the droplet flows through the portion in contact with a cooling zone is from 1 to 60%, preferably from 2 to 50%, more preferably from 5 to 40%, and most preferably from 10 to 30%.
